(19) Europäisches Patentamt
European Patent Office
Office européen des brevets

(11) Veröffentlichungsnummer: **0 459 198 A2**

(12) # EUROPÄISCHE PATENTANMELDUNG

(21) Anmeldenummer: **91107665.1**

(51) Int. Cl.5: **C07D 307/64**, A61K 31/34

(22) Anmeldetag: **11.05.91**

(30) Priorität: **23.05.90 DE 4016536**

(43) Veröffentlichungstag der Anmeldung: **04.12.91 Patentblatt 91/49**

(84) Benannte Vertragsstaaten: **CH DE FR GB IT LI NL**

(71) Anmelder: **HAARMANN & REIMER GMBH**
**Postfach 138**
**W-3450 Holzminden(DE)**

(72) Erfinder: **Emberger, Roland, Dr.**
**Bärenfang 4**
**W-3450 Holzminden(DE)**
Erfinder: **Güntert, Matthias, Dr.**
**Vogelsang 4**
**W-3450 Holzminden(DE)**
Erfinder: **Hopp, Rudolf, Dr.**
**Auf dem Gehrenkamp 28**
**W-3450 Holzminden(DE)**
Erfinder: **Köpsel, Manfred, Dr.**
**Schinkelstrasse 1**
**W-3450 Holzminden(DE)**
Erfinder: **Kuhn, Walter, Dr.**
**Hebbelstrasse 6**
**W-3450 Holzminden(DE)**
Erfinder: **Werkhoff, Peter, Dr.**
**Sieplerstrasse 24**
**W-3470 Höxter 1(DE)**

(74) Vertreter: **Petrovicki, Wolfgang, Dr. et al**
**Bayer AG Konzernverwaltung RP Patente Konzern**
**W-5090 Leverkusen 1 Bayerwerk(DE)**

(54) **Thio-alkanone, Verfahren zu ihrer Herstellung und ihre Verwendung.**

(57) Die Erfindung betrifft neue Thio-alkanone der Formel

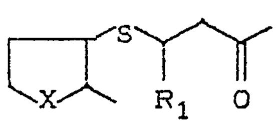

(I),

in der
X für ein Sauerstoff- oder Schwefelatom,
$R_1$ für Wasserstoff oder eine $C_1$-$C_2$-Alkylgruppe steht und die gestichelten Linien eine Einfachbindung oder keine Bindung darstellen,
ein Verfahren zu ihrer Herstellung und ihre Verwendung als Aromastoffe.

EP 0 459 198 A2

Die Erfindung betrifft neue Thio-alkanone, ein Verfahren zu ihrer Herstellung und ihre Verwendung als Aromastoffe.

Es wurden neue Thio-alkanone der Formel

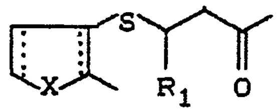

(I),

gefunden, in der

X für ein Sauerstoff- oder Schwefelatom,

$R_1$ für Wasserstoff oder eine $C_1$-$C_2$-Alkylgruppe steht und die gestrichelten Linien eine Einfachbindung oder keine Bindung darstellen.

Außerdem wurde gefunden, daß diese Thioalkanone der Formel (I) wertvolle organoleptische Eigenschaften aufweisen.

Die Erfindung betrifft daher die neuen Thioalkanone der Formel (I) und deren Verwendung als Aromastoffe.

Die erfindungsgemäßen Thioalkanone werden durch Addition von Thiolen der Formel

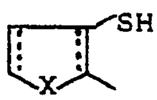

(II),

in der

X und die gestrichelten Linien die unter Formel (I) angegebene Bedeutung haben,

an Ketone der Formel

$$R_1-CH=CH-\overset{O}{\overset{\|}{C}}-CH_3 \qquad (III)$$

in der

$R_1$ die unter Formel (I) angegebene Bedeutung hat,

erhalten.

Die Erfindung betrifft daher auch ein Verfahren zur Herstellung von Thioalkanonen der Formel (I); das Verfahren ist dadurch gekennzeichnet, daß man Thiole der Formel (II) an Alkanone der Formel (III) addiert.

Die Addition kann bei Temperaturen von 0°C bis 50°C, vorzugsweise bei Raumtemperatur, in einem inerten Lösungsmittel wie Alkoholen, vorzugsweise Ethanol, oder Ethern, vorzugsweise Tetrahydrofuran, durchgeführt werden.

Die als Ausgangsverbindungen benötigten Thiole der Formel (II) sind in der DE-OS 24 58 609, die Ketone der Formel (III) z.B. in der DE-PS 877 606 und der DE-OS 3 212 326 beschrieben.

Die erfindungsgemäßen Verbindungen der Formel (I) sind wertvolle Aromastoffe; sie zeichnen sich durch sehr niedrige Geschmacksschwellenwerte aus. So fand eine Testpanel aus 20 Prüfern in einem Dreieckstest bei der Anwendung von 4-(2-Methyl-3-furyl-thio)-pentan-2-on in 0,5 %iger wäßriger Kochsalzlösung bereits einen signifikanten Unterschied zwischen der Null-Probe und der nur 1 ppb 4-(2-Methyl-3-furyl-thio)-pentan-2-on enthaltenden Kochsalzlösung. Für 4-(2-Methyl-3-tetrahydrofuryl-thio)-pentan-2-onfand ein Testpanel aus 6 speziell geschulten Prüfern einen signifikanten Unterschied zwischen der 0,5 %igen wäßrigen Kochsalzlösung und der 1,5 ppb 4-(2-Methyl-3-tetrahydrofuryl-thio)-pentan-2-on enthaltenden 0,5 %igen Kochsalzlösung.

Die Geschmacksbeschreibungen für die einzelnen erfindungsgemäßen Verbindungen der Formel (I) bei ihrer Anwendung in 0,5 %iger wäßriger Kochsalzlösung lauten:

4-(2-Methyl-3-furyl-thio)-pentan-2-on:

bei einem Zusatz von 15 ppb: Brühe, fleischig, Reaktionsaroma

bei einem Zusatz von 150 ppb: Fülle, fleischig, Rindfleischbrühe, leichte Bratennote im Hintergrund

4-(2-Methyl-3-tetrahydrofuryl-thio)-pentan-2-on:

bei einem Zusatz von 15 ppb: Kochfleisch, Fleischbrühe, leichte Bratennote

4-(2-Methyl-3-thienylthio)-pentan-2-on:

bei einem Zusatz von 15 ppb: pilzig, krautig

4-(2-Methyl-3-thienyl-thio)-butan-2-on:

bei einem Zusatz von 15 ppb: fettig, röstig, leicht Erdnuß, Fleisch

Mit ihrem spezifischen Geschmack in Richtung Fleisch wirken die erfindungsgemäßen Verbindungen der Formel (I) in Fleischaromakompositionen geschmacksverstärkend und -abrundend. Aber auch in anderen Aromakompositionen, z.B. Nußaromen, bewirken die erfindungsgemäßen Verbindungen eine Abrundung des Aromas und eine Erhöhung der Geschmacksfülle.

Die unter Verwendung der erfindungsgemäßen Verbindungen hergestellten Aromakompositionen können im gesamten Nahrungsmittel- und Genußmittelbereich, sowie in Tierfutter eingesetzt werden. Insbesondere sind sie geeignet für Fettmassen, Backwaren, Extrusionsprodukte, Fertiggerichte, Fleisch- und Wurstprodukte, Suppen, Soßen, Gemüsekonserven und alle Arten von industriell gefertigtem Tierfutter.

Die erfindungsgemäßen neuen Thio-alkanone werden in Mengen von 5 ppt bis 1 %, vorzugsweise 100 ppt bis 100 ppm, bezogen auf das verzehrfertige Nahrungsmittel verwendet.

Bei den in den Beispielen verwendeten Prozentangaben handelt es sich um Gewichtsprozente.

## Beispiel 1

10 g 2-Methylfuryl-3-thiol und 15 g 3-Penten-(2)-on wurden in 100 ml Ethanol gelöst. Die Lösung wurde 48 Stunden bei Raumtemperatur gelöst. Anschließend wurde das Lösungsmittel bei 50° C im Vakuum abdestilliert. Der Rückstand (18 g) wurde durch präparative Hochdruckflüssigkeitschromatographie (HPLC) gereinigt. Es wurden 9 g 4-(2-Methyl-3-furanyl-thio)-pentan-2-on (Reinheitsgrad 96 %) erhalten. IR-, NMR- und Massenspektren der Verbindung stimmen mit der für sie angegebenen Struktur überein.

Bei Verwendung von 2-Methyltetrahydrofuryl-3-thiol bzw. 2-Methyl-thienyl-3-thiol anstelle von 2-Methylfuryl-3-thiol und von 3-Buten-2-on anstelle von 3-Penten-2-on wurden 4-(2-Methyl-3-tetrahydrofuryl-thio)-pentan-2-on, 4-(2-Methyl-3-thienyl-thio)-pentan-2-on und 4-(2-Methyl-3-thienyl-thio)-butan-2-on erhalten.

## Beispiel 2

Eine Fleischaromakomposition wurde durch Mischen folgender Bestandteile in den angegebenen Gewichtsteilen hergestellt:

| | |
|---|---|
| 50:50-Mischung von Na-Inosinat und Na-Guanilat | 1 |
| Mononatriumglutamat | 19 |
| Milchsäure, sprühgetrocknet | 30 |
| Pflanzenproteinhydrolysat (Type RFB der FIS) | 350 |
| Süßmolkepulver | 100 |
| Speisesalz | 500 |
| | 1000 |

Eine 1 %ige wäßrige Lösung dieser Komposition diente als Kontrollprobe.

Wurde die Kontrollprobe mit 1,5 ppm 4-(2-Methyl-3-furyl-thio)-pentan-2-on versetzt, so wurde der Geschmack der wäßrigen Lösung von einer Testgruppe im Vergleich zur Kontrollprobe als deutlich voller und fleischiger in Richtung Rinderbraten beschrieben.

Bei Zugabe von 1,5 ppm 4-(2-Methyl-tetrahydrofuryl-thio)-pentan-2-on wurde der Geschmack als wesentlich voller mit ausgeprägt fleischigem Charakter und leichter Braten-(krusten)-note beschrieben.

## Patentansprüche

1. Thio-alkanone der Formel

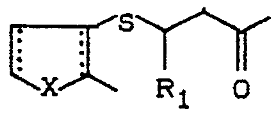

(I),

in der

X für ein Sauerstoff- oder Schwefelatom,

$R_1$ für Wasserstoff oder eine $C_1$-$C_2$-Alkylgruppe steht und die gestrichelten Linien eine Einfachbindung oder keine Bindung darstellen.

2. 4-(2-Methyl-3-furyl-thio)-pentan-2-on.

3. 4-(2-Methyl-3-tetrahydrofuryl-thio)-pentan-2-on.

4. Verfahren zur Herstellung von Thioalkanonen der Formel (I), dadurch gekennzeichnet, daß man Thiole der Formel

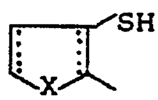

(II),

in der

X und die gestrichelten Linien die unter Formel (I) angegebene Bedeutung haben,

an Ketone der Formel

$$R_1-CH=CH-\overset{O}{\overset{\|}{C}}-CH_3 \qquad (III)$$

in der

$R_1$ die unter Formel (I) angegebene Bedeutung hat,

addiert.

5. Verwendung der Thio-alkanone nach Anspruch 1 als Aromastoffe.